# EUROPEAN PATENT SPECIFICATION

(11) **EP 0 560 207 B1**
(45) Date of publication and mention of the grant of the patent: **07.07.1999**
(21) Application number: 93103467.2
(22) Date of filing: 04.03.1993
(51) Int. Cl.: A61K 31/19, A61K 31/44, A61K 9/00, A61K 47/22

(54) **Composition based on ibuprofen, for oral usage**
Oral anzuwendendes Arzneimittel bestehend aus Ibuprofen
Composition orale à base d'ibuprofène

(30) Priority: 12.03.1992 IT RM920173
(43) Date of publication of application: 15.09.1993
(73) Proprietor: AZIENDE CHIMICHE RIUNITE ANGELINI FRANCESCO A.C.R.A.F. S.p.A., 00181 Roma (IT)
(72) Inventor: Depalmo, Galli Angeli, I-60015 Falconara Marittima (IT)
(74) Representative: Marchi, Massimo, Dr.

(56) References cited:
- US-A- 4 564 614

## Description

The present invention relates to a novel formulation of ibuprofen, suitable in order to deliver said drug to humans or animals, by oral way and as a solution.

Ibuprofen, i.e., 2-methyl-2-(4-isobutyl-phenyl)-acetic acid was disclosed first in U.K. patent No. 971 700 issued on Sept. 30th, 1964, and was adopted for therapeutical use, as an antirheumatic drug.

However, it was very soon possible to appreciate the considerable value thereof also as an anti-inflammatory analgesic drug for very widely usage, which displays, as compared to the well-know drugs presently known for such an application, such as, e.g., acetylsalicylic acid, commonly known as "aspirin", the advantage of a lower gaster aggressivity.

Of course, a large number of pharmaceutical formulations were proposed in the past and were drawn on the market, which contain ibuprofen, either as the only active principle, or associated with other active principles. So, ibuprofen is administered by oral way as tablets, sugar-coated pills, ready-to-use suspensions, or extemporaneous suspensions.

Furthermore, suppositories, solutions and creams and salves, were introduced and used to respectively administer the drug by rectal way and by parenteral way; and for topic application.

In the preparation of some of these formulations, ibuprofen is used not in its free-acid form, but as a salt with an either organic or inorganic base; so, e.g., its lisine salt in salves, injections, suppositories and tablets (Aciril®, Arfen®); its methyl-glucamine salt in salves, injections and tablets (Artrene®); and its sodium salt (Brufen® suppositories); were described and traded, at least in Italy.

Those skilled in the art are also well aware of the fact that, for the pharmaceutical forms to be administered by oral way, with the other conditions being the same, the solutions -- when, as it occurs in the specific case of ibuprofen, the chemical stability of the product so allows -- have to be preferred over suspensions, both because the solutions lead to a faster and more constant absorption and because, from a physical view point, they result to remain much more homogeneous than suspensions over time, and after undergoing any possible fortuitous stresses. In US-A-4 564 614 there is disclosed the use of ibuprofen and pyridoxine as active principles, but they are used in a solid phase (tablets/capsules) and for treating inflammation in a mammal.

Now, as far as we know, it does not result to us that liquid pharmaceutical forms have ever been used which contain ibuprofen as a solution, and are designed in order to deliver said drug by oral way for therapeutical treatment of humans and animals.

A careful study of specialized scientific literature has furthermore shown that such a situation is due to the extremely unpleasant flavour of ibuprofen, and its salts. In fact, taking per os an aqueous suspension of ibuprofen, or, even worse, an aqueous solution of an ibuprofen salt, causes an intense heat burn feeling in the patient's throat, followed, after a short time, by an intense and long-lasting bitter aftertaste, which results impossible to be masked by the commonly used pharmaceutically acceptable excipients and sweeteners.

Different approaches were attempted in order to solve this technical problem; so, e.g., already in 1977, A.A. Sinkula obtainded a patent (U.S. patent No. 4,049,699), in which he claims the semicarbazone of ibuprofen ester with p-hydroxybenzaldehyde, prepared with the declared purpose of masking the taste of ibuprofen.

More recently, in an attempt to solve the problem of ibuprofen taste, a salt of ibuprofen with N-methylglucamine was disclosed (U.S. patent 5,028,625), the use of which is anyway claimed for the only preparation of chewable tablets.

Unexpectedly, the present Applicant found now that an aqueous solution, which may even be a concentrated one, of an ibuprofen salt -- in particular, of potassium ibuprofen salt -- can be made palatable when it contains a certain amount of Vitamin B6 (pyridoxine), or of a salt thereof. After a number of trials, the present Applicant was able to establish that the amount of Vitamin B6 should be comprised within the approximate range of from 1/8 to 1/2 content of ibuprofen in its free acid form. In particular, the optimal amount of Vitamin B6 or of a salt thereof is of approximately 1/4 of the content of ibuprofen in free acid form. Smaller amounts (e.g., 1/8) lead still to a detectable improvement in product palatability, even if not to an optimal extent.

Amounts of Vitamin B6 larger than 1/2 content of ibuprofen in free acid form, by weight, supply the compositions with an additional pharmacologic effect, which generally is undesired.

A solution prepared according to the present invention does not cause, per se, a heat burn feeling in user's throat, or a bitter aftertaste, but does not display a particularly pleasant taste. In order that the end composition displays pleasant smell and taste characteristics, sweeteners and flavouring agents should be added, as in any other pharmaceutical forms.

Therefore, the subject-matter of the present invention is a pharmaceutical composition for oral administration of ibuprofen as the active principle, which comprises an aqueous solution of (a) pyridoxine or of a pharmaceutically acceptable salt thereof, in a ratio comprised within the range of from about 1/8 to about 1/2 by weight to the weight of ibuprofen as ibuprofen in free acid form, and (b) a therapeutical acceptable ibuprofen salt, together with suitable pharmaceutically inert excipients.

A particularly preferred pharmaceutical composition according to the present invention is that composition which contains pyridoxine or a salt thereof in a ratio of approximately 1/4 by weight, to the weight of ibuprofen as ibuprofen in its free acid form.

The expression "pharmaceutically acceptable salts" encompasses according to the present invention all those pharmaceutically acceptable salts which are used in therapeutical applications of ibuprofen and pyridoxine.

In particular, in the composition according to the present invention, the potassium salt of ibuprofen and pyridoxine hydrochloride are preferred.

When for the pharmaceutical form according to the present invention pyridoxine hydrochloride is used, also a suitable amount of a basic substance should be added, such as to neutralize the hydrochloric acid contained in said salt, which hydrochloric acid might endanger the stability of the products in solution.

By the expression "pharmaceutically inert excipients", those sweeteners, stabilizers, preservatives, and so forth, are encompassed, which are commonly used for suitable preparations for oral administration in syrup form.

The following examples are supplied for the purpose of illustrating the invention in greater detail.

### Example 1

### Drops at 20%

| 100 ml of drops contain: | |
|---|---|
| Ibuprofen | 20.0 g |
| Potassium hydroxide | 7.07 g |
| Glycerol O.P.* | 15.0 g |
| Pyridoxine hydrochloride | 5.0 g |
| Sodium EDTA | 0.01 g |
| Sodium sulfite | 0.2 g |
| Methyl p-hydroxybenzoate | 0.147 g |
| Propyl p-hydroxybenzoate | 0.0315 g |
| Sodium citrate . 2H₂O | 1.5 g |
| Saccharin sodium | 1.5 g |
| Refined sugar | 20.0 g |
| Flavour | 11.0 g |
| Demineralized water q.s. to 100 ml. | |

| | |
|---|---|
| * O.P. = Official Pharmacopoeia | |

On considering that ibuprofen formulated for administration by oral way is generally available in such unit dosage forms as sugar-coated pills or bars containing from 200 to 600 mg, and that 10 drops of the above solution contain 100 mg of ibuprofen, said drops should be diluted in a suitable liquid, such as, e.g., water, in such a number as to reach the most suitable dosage as required in each specific case.

In practice, the use of such drops will be particularly convenient for those indications in which ibuprofen is used at lob dosage rates, such as, e.g., in various kinds of pain from various origins.

### Example 2

### Syrup at 4%

| 100 ml of syrup contain: | |
|---|---|
| Ibuprofen | 4.0 g |
| Potassium hydroxide | 1.4 g |
| Glycerol O.P. | 3.0 g |
| Pyridoxine hydrochloride | 1.0 g |
| Sodium EDTA | 0.01 g |
| Sodium sulfite | 0.2 g |
| Methyl p-hydroxybenzoate | 0.15 g |
| Propyl p-hydroxybenzoate | 0.03 g |
| Sodium citrate . 2H₂O | 3.0 g |
| Saccharin sodium | 0.5 g |
| Refined sugar | 20.0 g |
| Flavour | 2.2 g |
| Demineralized water q.s. to 100 ml. | |

A spoonful (15 ml) of this syrup contains 600 mg of ibuprofen. This syrup is hence particularly suitable for the administration of ibuprofen in those indications in which, as in osteoarthrosis, said drug is used at high dosage rates.

## Claims

1. A pharmaceutical composition for oral administration of ibuprofen as the active principle, characterized in that said composition contains an aqueous solution of (a) pyridoxine or of a pharmaceutically acceptable salt thereof, in a ratio comprised within the range of from 1/8 to 1/2 by weight to the weight of ibuprofen as ibuprofen in its free acid form, and (b) a therapeutically acceptable ibuprofen salt, together with suitable pharmaceutically inert excipients.

2. A pharmaceutical composition according to claim 1, characterized in that pyridoxine or a salt thereof is contained in a ratio of 1/4 by weight, to the weight of ibuprofen as ibuprofen in its free acid form.

3. A pharmaceutical composition according to claims 1 and 2, characterized in that said ibuprofen salt is ibuprofen potassium salt.

4. A pharmaceutical composition according to anyone of claims from 1 to 3, characterized in that said pyridoxine salt is pyridoxine hydrochloride, in the presence of such an amount of a basic substance, as a further component of the solution to neutralize also the hydrochloric acid contained in said salt.

5. A pharmaceutical composition according to anyone of claims from 1 to 4, for oral administration as drops.

6. A pharmaceutical composition according to anyone of claims from 1 to 4, for oral administration as a syrup.

## Patentansprüche

1. Pharmazeutische Zubereitung zur oralen Verabreichung von Ibuprofen als Wirkprinzip, dadurch gekennzeichnet, daß diese Zubereitung in wässeriger Lösung folgende Bestandteile enthält:
(a) Pyridoxin oder ein pharmazeutisch akzeptables Pyridoxin-Salz in einer solchen Menge, daß das Gewichtsverhältnis *Pyridoxin beziehungsweise pharmazeutisch akzeptables Pyridoxin-Salz/Ibuprofen in Form der freien Säure* 1/8 bis 1/2 beträgt,
und
(b) ein therapeutisch akzeptables Ibuprofen-Salz in Kombination mit geeigneten pharmazeutisch inerten Grundstoffen.

2. Pharmazeutische Zubereitung nach Anspruch 1, dadurch gekennzeichnet, daß sie Pyridoxin oder ein Pyridoxin-Salz in einer solchen Menge enthält, daß das Gewichtsverhältnis *Pyridoxin beziehungsweise Pyridoxin-Salz/Ibuprofen in Form der freien Säure* 1/4 beträgt.

3. Pharmazeutische Zubereitung nach Anspruch 1 oder 2, dadurch gekennzeichnet, daß besagtes Ibuprofen-Salz das Kaliumsalz des Ibuprofens ist.

4. Pharmazeutische Zubereitung nach einem der Ansprüche 1 bis 3, dadurch gekennzeichnet, daß besagtes Pyridoxin-Salz Pyridoxin-hydrochlorid ist, das in Gegenwart einer solchen Menge einer basischen Substanz als ein weiterer Bestandteil der Lösung vorliegt, die ausreicht, um auch die in diesem Salz enthaltene Chlorwasserstoffsäure zu neutralisieren.

5. Pharmazeutische Zubereitung nach einem der Ansprüche 1 bis 4 zur oralen Verabreichung in Form von Tropfen.

6. Pharmazeutische Zubereitung nach einem der Ansprüche 1 bis 4 zur oralen Verabreichung in Form eines Sirups.

## Revendications

1. Composition pharmaceutique pour administration orale d'ibuprofène en tant que principe actif, caractérisée en ce que ladite composition contient une solution aqueuse (a) de pyridoxine ou d'un sel acceptable d'un point de vue pharmaceutique de cette dernière, selon un rapport compris entre 1/8 et 1/2 par rapport au poids de l'ibuprofène sous sa forme acide libre, et (b) d'un sel d'ibuprofène acceptable d'un point de vue thérapeutique, en même temps que des excipients appropriés, inertes d'un point de vue pharmaceutique.

2. Composition pharmaceutique selon la revendication 1, caractérisée en ce que la pyridoxine ou un sel de cette dernière est présente selon un rapport de 1/4 à la totalité en poids par rapport à l'ibuprofène sous sa forme acide libre.

3. Composition pharmaceutique selon la revendication 1 ou 2, caractérisée en ce que ledit sel d'ibuprofène est le sel de potassium de l'ibuprofène.

4. Composition pharmaceutique selon l'une quelconque des revendications 1 à 3, caractérisée en ce que ledit sel de pyridoxine est le chlorhydrate de pyridoxine, en présence d'une quantité d'une substance basique, en tant que composant supplémentaire de la solution, suffisante pour neutraliser aussi l'acide chlorhydrique contenu dans ledit sel.

5. Composition pharmaceutique selon l'une quelconque des revendications 1 à 4, pour administration orale sous forme de gouttes.

6. Composition pharmaceutique selon l'une quelconque des revendications 1 à 4, pour administration orale sous forme d'un sirop.
